# EUROPÄISCHE PATENTSCHRIFT

(11) **EP 2 173 700 B1**
(45) Veröffentlichungstag und Bekanntmachung des Hinweises auf die Patenterteilung: **28.01.2015**
(21) Anmeldenummer: 08760880.8
(22) Anmeldetag: 12.06.2008
(51) Int. Cl.: C07C 51/15, C07C 59/185, C07C 323/52, C07C 51/373, C07C 67/313, C07D 317/32, C07D 493/08, C07C 319/20, C07C 319/14, C07B 41/08, C07B 41/06

(54) **VERFAHREN ZUR HERSTELLUNG VON KETOSÄUREN UND DEREN DERIVATEN**
METHOD FOR THE PRODUCTION OF KETOACIDS AND THEIR DERIVATIVES
PROCÉDÉ DE FABRICATION DE CÉTOACIDES ET LEURS DÉRIVÉS

(30) Priorität: 09.07.2007 DE 102007031917
(43) Veröffentlichungstag der Anmeldung: 14.04.2010
(73) Patentinhaber: Evonik Degussa GmbH, 45128 Essen (DE)
(72) Erfinder: KOBLER, Christoph, 63755 Alzenau (DE); HATELEY, Martin, 81825 München (DE); ROTH, Philipp, 63456 Hanau (DE); JÄGER, Barbara, 63533 Mainhausen (DE); WECKBECKER, Christoph, 63584 Gründau-Lieblos (DE); HUTHMACHER, Klaus, 63571 Gelnhausen (DE)
(86) Internationale Anmeldenummer: PCT/EP2008/057334
(87) Internationale Veröffentlichungsnummer: WO 2009/007193

(56) Entgegenhaltungen:
- WO-A-2006/072711
- US-A- 5 627 185
- TROST B, SALZMANN T N: "Applications of Sulfenylations of Ester Enolates. Synthesis of Pheromones of the Honey Bee" J ORG CHEM, Bd. 40, Nr. 1, 1975, Seiten 148-150, XP002504043
- A I MEYERS ET AL.: "The Use of Dithioesters as Acyl Anion Equivalents. Double Homologation of the "CH2-C=O" Synthon" TETRAHEDRON LETTERS, Bd. 47, 1978, Seiten 4657-4660, XP002504044
- DATABASE BEILSTEIN BEILSTEIN INSTITUTE FOR ORGANIC CHEMISTRY, FRANKFURT-MAIN, DE; XP002504046 gefunden im XFIRE Database accession no. BRN 1729347 & LUND 1929, 1929, Seiten 109-110,
- DATABASE BEILSTEIN BEILSTEIN INSTITUTE FOR ORGANIC CHEMISTRY, FRANKFURT-MAIN, DE; XP002504047 gefunden im XFIRE Database accession no. brn 2536576 & CHEMISCHE BERICHTE, Bd. 36, 1903, Seite 298,
- DATABASE BEILSTEIN BEILSTEIN INSTITUTE FOR ORGANIC CHEMISTRY, FRANKFURT-MAIN, DE; XP002504048 gefunden im XFIRE Database accession no. brn 6393736 & BULLETIN OF THE ACADEMY OF SCIENCES OF THE USSR, DIVISION OF CHEMICAL SCIENCE, Bd. 32, Nr. 1, 1983, Seiten 186-189,
- DATABASE BEILSTEIN BEILSTEIN INSTITUTE FOR ORGANIC CHEMISTRY, FRANKFURT-MAIN, DE; XP002504049 gefunden im XFIRE Database accession no. brn 1937904 & TETRAHEDRON LETTERS, 1978, Seite 5021,
- DATABASE BEILSTEIN BEILSTEIN INSTITUTE FOR ORGANIC CHEMISTRY, FRANKFURT-MAIN, DE; XP002504050 gefunden im XFIRE Database accession no. brn 1944840 & TETRAHEDRON LETTERS, 1978, Seiten 5657-4660,
- DATABASE BEILSTEIN BEILSTEIN INSTITUTE FOR ORGANIC CHEMISTRY, FRANKFURT-MAIN, DE; XP002504051 gefunden im XFIRE Database accession no. brn 1937791 & TETRAHEDRON LETTERS, 1979, Seiten 4657-4660,
- DATABASE BEILSTEIN BEILSTEIN INSTITUTE FOR ORGANIC CHEMISTRY, FRANKFURT-MAIN, DE; XP002504052 gefunden im XFIRE Database accession no. brn 1863371 & CANADIAN JOURNAL OF CHEMISTRY, Bd. 58, 1980, Seiten 716-722,
- DATABASE BEILSTEIN BEILSTEIN INSTITUTE FOR ORGANIC CHEMISTRY, FRANKFURT-MAIN, DE; XP002504053 gefunden im XFIRE Database accession no. brn 5600320 & CANADIAN JOURNAL OF CHEMISTRY, Bd. 58, 1980, Seiten 716-722,
- DATABASE BEILSTEIN BEILSTEIN INSTITUTE FOR ORGANIC CHEMISTRY, FRANKFURT-MAIN, DE; XP002504054 gefunden im XFIRE Database accession no. brn 1772265 & CHEMISCHE BERICHTE, Bd. 32, 1899, Seite 2808,
- CREGGE ET AL.: "A Versatile and Reactive Michael Receptor for the Synthesis of 1,4-Dicarbonly Compounds" TETRAHEDRON LETTERS, Bd. 28, 1973, Seiten 2603-2606, XP002504045
- DATABASE BEILSTEIN BEILSTEIN INSTITUTE FOR ORGANIC CHEMISTRY, FRANKFURT-MAIN, DE; XP002504055 gefunden im XFIRE Database accession no. reaction 3780578 & CANADIAN JOURNAL OF CHEMISTRY, Bd. 58, 1980, Seiten 716-722,
- DATABASE BEILSTEIN BEILSTEIN INSTITUTE FOR ORGANIC CHEMISTRY, FRANKFURT-MAIN, DE; XP002504056 gefunden im XFIRE Database accession no. brn 1765525, reaction 648735 & HOPPE SEYLER'S ZEITSCHRIFT FÜR PHYSIOLOGISCHE CHEMIE, Bd. 16, 1892, Seite 576,
- DATABASE BEILSTEIN BEILSTEIN INSTITUTE FOR ORGANIC CHEMISTRY, FRANKFURT-MAIN, DE; XP002504057 gefunden im XFIRE Database accession no. reaction 4688389 & BIORG MED CHEM, Bd. 5, Nr. 4, 1997, Seiten 707-714,
- QASMI D ET AL: "SYNTHESIS OF N-GLYOXYLYL PEPTIDES AND THEIR IN VITRO EVALUATION AS HIV-1 PROTEASE INHIBITORS" BIOORGANIC & MEDICINAL CHEMISTRY, ELSEVIER SCIENCE LTD, GB, Bd. 5, Nr. 4, 1. Januar 1997 (1997-01-01), Seiten 707-714, XP000889656 ISSN: 0968-0896

## Beschreibung

Die vorliegende Erfindung bezieht sich auf ein neues Verfahren zur Herstellung von α-Ketosäuren, insbesondere von α-Ketomethionin, und deren Derivaten.

α-Ketosäuren sind wichtige Produkte und werden unter anderem für die Supplementierung von Aminosäuren in der Therapie von chronischem Nierenversagen verwendet (Jungers et al., Blood Purification 1988, 6, 299-314 und Clasen et al., Med. Klin. 1978, 73, 1403-1408).

Die Synthese von α-Ketosäuren ist auf verschiedene Art und Weise in der Literatur beschrieben, unter anderem auch durch die Reaktion von einem Grignardreagenz mit einem Dialkyloxalat und anschließender Hydrolyse des entstandenen Esters zur freien Säure (Rambaud et al., Synthesis 1988, 564 und Macritchie et al., Tetrahedron: Asymmetry 1997, 8, 3895). Ebenfalls liefert die saure katalysierte Hydrolyse von Acylcyaniden α-Ketosäuren (Nozaki et al., Tetrahedron: Asymmetry 1993, 4, 2179). Billek berichtete über die Herstellung einer Reihe von α-Ketosäuren. Dabei werden Alkyliden-Hydantoine unter basischen Reaktionsbedingungen verseift (Billek, Monath. Chem. 1961, 92, 343-352).

α-Ketosäuren können auch aus dem entsprechenden Aldehyd und Kohlendioxid durch eine Umpolungsreaktion hergestellt werden. Eine Möglichkeit zur Umpolung des Aldehyds stellt die Bildung eines cyclischen Dithians dar, aus dem ein Carbanion durch Deprotonierung mit einer starken Base gebildet werden kann. Dieses Carbanion kann anschließend mit Kohlendioxid umgesetzt werden. Das cyclische Dithian wird abschließend unter oxidativen Bedingungen z.B. mit Quecksilbersalzen zu der gewünschten α-Ketosäure gespalten.

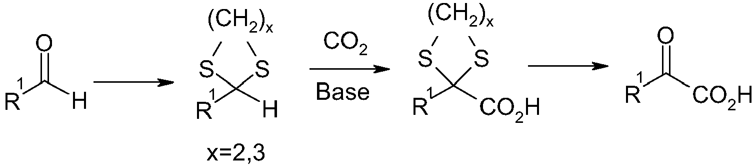

Nach Arbeiten von Corey und Seebach [Corey et al., Angew. Chem. 1965, 77, 1134-1136 und Seebach et al., J. Org. Chem. 1975, 40, 231-237] werden üblicherweise Dithiole verwendet, um cyclische Dithioacetale (Dithiane) zu synthetisieren. Dies gelingt besonders leicht, da die Bildung von cyclischen Dithianen aufgrund der hohen thermodynamischen Stabilität des gebildeten Fünf- bzw. Sechsrings bevorzugt abläuft. Die Cyclisierung mit Dithiolen ist zudem kinetisch bevorzugt. Genau diese hohe Stabilität stellt jedoch einen gravierenden Nachteil dar, da die Spaltung des Dithianrings dadurch extrem erschwert wird und damit die Freisetzung der gewünschten α-Ketosäure nur unter chemisch drastischen Bedingungen gelingt. Die Spaltung des Dithians wird normalerweise durch einen Oxidationsprozess herbeigeführt [Seebach, Synthesis 1969, 17-36]. Damit sind eine Abtrennung und ein Wiedereinsetzen des Dithiols nicht mehr möglich, da der Schwefel zur Abtrennung oxidiert und anschließend mit Quecksilbersalzen als schwerlösliches Salz gefällt wird. Weitere Nachteile dieses Verfahrens sind die hohen Kosten von geeigneten Dithiolen wie z.B. 1,2-Ethandithiol oder 1,3-Propandithiol sowie die kleinen Mengen, die für eine großtechnische Anwendung verfügbar wären. Diese Probleme machen die industrielle Anwendung der oben beschriebenen Umpolungsmethode sehr unattraktiv.

In der Literatur sind mehrere Beispiele zur Umsetzung von kurzkettigen Thiolen wie z.B. Methylmercaptan mit Aldehyden zu den entsprechenden Thioacetalen beschrieben [Trofimov et al., J. Org. Chem. USSR 1972, 8, 2036 und Rothstein et al. J. Chem. Soc. 1940, 1563]. Zudem ist die Verwendung von diesen Dithioacetalen in Umpolungsreaktionen mit verschiedenen Elektrophilen bekannt. Es gibt jedoch nur ein einziges Literaturbeispiel, wo ein aromatisches Thioacetal mit CO₂ in einer Umpolungsreaktion erfolgreich umgesetzt wurde [Micetich et al., Heterocycles 1985, 23, 585-592].

α-Ketomethionin stellt eine besondere α-Ketosäure dar, da sie intermediär im Organismus bei der Umwandlung von D-Methionin in L-Methionin gebildet wird. α-Ketomethionin ist durch zahlreiche Syntheserouten erhältlich und in der Literatur beschrieben. Zudem sind einige Salze und andere Derivate wie z. B. Ketomethionin Ester literaturbekannt. Die Verfahren zur Darstellung von α-Ketomethionin kann man dabei grundsätzlich in chemische und biochemische Verfahren aufteilen:

### a) Biochemische Synthesen:

Meister erhielt das Natriumsalz von α-Ketomethionin in einer Ausbeute von 77% durch die L-Aminooxidasen katalysierte Oxidation von Methionin (Meister, J. Biol. Chem. 1952, 197, 309). Zuvor zeigte Waelsch et al., dass, die in der Leber enthaltenen Aminooxidasen, Methionin zu α-Ketomethionin umwandeln können (Waelsch et al., J. Am. Chem. Soc. 1938, 61, 2252).

Mosbach et al. beschreibt ebenfalls die Herstellung von α-Ketomethionin durch die von L-Aminooxidasen katalytisierte Oxidation von Methionin. Dabei werden immobilisierte Providencia sp. PCM 1298 Zellen verwendet (Mosbach et al. Enzyme Microb. Technol. 1982, 4, 409).

Die Nachteile der Herstellung von α-Ketomethionin bzw. dessen Derivaten mit Hilfe biologischer Systeme, entweder unter Verwendung gereinigter Enzyme oder ganzer Zellen, sind meist die niedrigeren Raum-Zeit Ausbeuten sowie die technisch aufwendige Isolation und Produktreinigung. Bei der Verwendung von hochreinen Enzymen kommt hinzu, dass die Entwicklung, Produktion und Aufreinigung der Enzyme sehr teuer und kompliziert ist und die Wiederverwendung bereits eingesetzter Enzyme meist nicht möglich ist.

### b) Chemische Synthesen:

Sakurai et al. veröffentlichten 1957 eine erste chemische Syntheseroute zur Darstellung von α-Ketomethionin. Dabei wurde als Schlüsselschritt Methyl-α-methoxalyl-γ-methylmercaptopropionat mit verdünnter Salzsäure zu α-Ketomethionin hydrolysiert (Sakurai et al., J. Biochem. 1957, 44, 9, 557).

Yamada et al. publizierten fast zeitgleich dieselbe Syntheseroute, nachdem erste Versuche zur Darstellung von α-Ketomethionin über einen intermediär gebildeten α-Oximoester lediglich niedrigere Ausbeuten lieferten (Chibata et al., Bull. Agr. Chem. Soc. Japan 1957, 21, 336).

Das von Sakurai und Yamada veröffentlichte Verfahren hat den großen Nachteil, dass erhebliche Salzmengen gebildet werden, die eine großtechnische Umsetzung unmöglich machen. Zudem ist die Syntheseroute nicht atomökonomisch, da ein Teil des Moleküls in einem Syntheseschritt als Kohlendioxid abgespalten und damit verloren geht. Daher wäre eine großtechnische Umsetzung dieser Syntheseroute zu teuer und nicht wirtschaftlich.

In der Patentanmeldung WO 06-72711 wurde vor kurzem die Herstellung von α-Ketomethionin ausgehend von Butadien veröffentlicht. Dabei wird Butadien selektiv zum ungesättigten Monoepoxid oxidiert und anschließend zum entsprechenden 1,2-Diol durch eine sauer-katalysierte Ringöffnung mit Wasser umgesetzt. Die folgende Oxidation des 1,2-Diols zur α,β-ungesättigten-α-Ketosäure und die darauf folgende 1,4-Addition von MeSH führt zu α-Ketomethionin.

Dieses Verfahren hat den Nachteil, dass teures Butadien als Edukt benötigt wird. Es ist wahrscheinlich, dass der Butadienpreis in den kommenden Jahren deutlich in Abhängigkeit zum Erdölpreis weiter steigen wird. Ein weiterer Nachteil, des in der WO 06-727211 beschriebenen Prozesses, ist die Tatsache, dass keine der bereits bestehenden Anlagen zur Methionin- bzw. MHA-Produktion verwendet werden könnte und daher neue Produktionsanlagen für jeden einzelnen beschriebenen Prozessschritt gebaut werden müssten. Die in der WO 06-72711 beschriebene Syntheseroute führt immer zum freien α-Ketomethionin, welches instabil und sehr schwierig zu isolieren ist.

α-Ketomethionin bzw. dessen Derivate, wie z. B. dessen Salze oder Ester, sind besonders wichtige Verbindungen, da sie Alternativen zu Methionin bzw. zum Methionin-Hydroxy-Analog (MHA) als Futtermitteladditive darstellen.

Rudolph et al. berichteten 1942, dass das Natriumsalz von α-Ketomethionin als Ersatz für D,L-Methionin eingesetzt werden kann und dadurch das Wachstum junger Ratten beschleunigt wird (Rudolph et el., J. Biol. Chem. 1942, 145, 210).

Es konnte mehrmals gezeigt werden, dass α-Ketomethionin den benötigten Schwefel für die Biosynthese von L-Methionin und L-Cystein liefern kann. (Sizer et al., Poultry Sci. 1964, 44, 673; Baker et al., Poultry Sci. 1975, 54, 584 und Baker, J. Nutr. 1976, 106, 1376).

Darüber hinaus konnten Baker und Harter zeigen, dass das Calciumsalz von α-Ketomethionin eine relative Biowertigkeit bezüglich des Wachstums von Hühnern von 83% im Vergleich zu L-Methionin (100%) und MHA (53%) besitzt (Baker and Harter, Proceedings of the Society for Experimental Biology and Medicine 1977, 156, 201). Die relative Biowertigkeit von verschiedenen Methioninderivaten, darunter α-Ketomethionin (90% im Vergleich zu L-Methionin) wurde von Baker in "Utilization of Precursors for L-Amino Acids" auf Seite 39 veröffentlicht.

Die Verwendung von α-Ketomethionin und von dessen Salzen und Estern und Amiden als Futtermitteladditiv ist in der WO 06-72711 beschrieben.

Vor dem Hintergrund der Nachteile des Standes der Technik ist es die Aufgabe, ein verbessertes, umweltfreundlicheres und groß-technisch realisierbares Verfahren zur Herstellung von Ketosäuren, insbesondere von α-Ketomethionin und dessen Derivaten als Alternative zu Methionin bzw. MHA als Futtermitteladditive, bereit zustellen.

Diese Aufgabe wird durch das erfindungsgemäße Verfahren gelöst.

Gegenstand der Erfindung ist ein Verfahren zur Herstellung von α-Ketosäuren sowie deren Derivaten der allgemeinen Formel (I) oder (II), 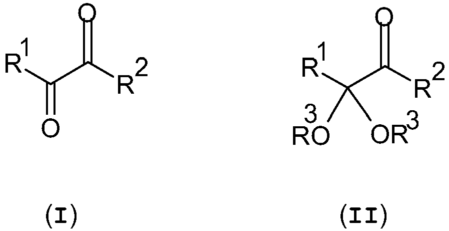 worin
R¹ eine verzweigte oder geradkettige C₁-C₁₈-Alkyl-, C₅-C₈-Cycloalkyl-, Vinyl-,
Allyl-, C₆-C₁₀-Aryl-C₁-C₄-alkylen-, vorzugsweise Benzyl-, oder C₄-C₉-Heteroaryl-C₁-C₄-alkylen-Gruppe, vorzugsweise 4-Imidazolylmethylen- oder 3-Indolylmethylen-, bedeutet, wobei R¹ gegebenenfalls substituiert ist,
R² -OR''' oder -NR'R'' bedeutet, worin R' und R'' gleich oder verschieden sein können und ein Wasserstoffatom oder eine verzweigte oder geradkettige C₁-C₆-Alkylgruppe bedeuten, und R''' ein Wasserstoffatom, eine verzweigte oder geradkettige C₁-C₈-Alkyl-, C₅-C₈-Cycloalkyl-, Allyl- oder eine Benzylgruppe bedeutet, wobei Alkyl in R', R" und/oder R"' gegebenenfalls substituiert ist, oder R''' ein Alkali-, Erdalkali- oder ein ein- oder zweiwertiges Übergangsmetallion bedeutet,
R³ gleich oder verschieden ist und ein Wasserstoffatom, eine verzweigte oder geradkettige C₁-C₈-Alkyl-, Allyl- oder eine Benzylgruppe bedeutet, wobei Alkyl und/oder Benzyl gegebenenfalls substituiert ist, oder die beiden Reste R³ zusammen C₂-C₈-Alkandiyl sind und gemeinsam einen Ring bilden, oder die beiden Reste R³ und R"' zusammen Teil einer C₃-C₈-Alkantriylgrupe der allgemeinen Formel -R³(CH-)R"'-sind und gemeinsam eine bicyclische Gruppe bilden,
wobei
   a) ein Aldehyd R¹CHO mit zwei gleichen oder verschiedenen Thiolen der Formel R⁴-SH, worin R⁴ eine verzweigte oder geradkettige, gegebenenfalls substituierte C₁-C₆-Alkyl-, C₅-C₈-Cycloalkyl-, Allyl- oder Benzylgruppe ist, zum entsprechenden Dithioacetal umgesetzt wird,
   b) das gebildete Dithioacetal mit carbonylhaltigen Elektrophilen wie Kohlendioxid, Phosgen, Chlorameisensäureester, Orthoameisensäureester oder Dialkylcarbonat in Gegenwart einer starken Base und nach Hydrolyse zur α,α-(Dithio)carbonsäure oder deren Derivaten der Formel (III) 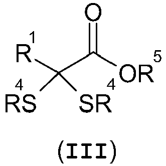 reagiert, worin R⁵ ein Wasserstoffatom oder eine verzweigte oder geradkettige C₁-C₆-Alkylgruppe bedeuten kann und die Reste R⁴ und R¹ die oben genannte Bedeutung haben, und
   c) die α,α-(Dithio)carbonsäure oder deren Derivate der Formel (III) durch säurekatalysierte Solvolyse in Gegenwart von mindestens 1 Moläquivalent Wasser unter Freisetzung von Thiolen der Formel R⁴SH zur α-Ketosäure oder deren Derivaten der allgemeinen Formel (I) oder (II) umgesetzt wird.

Die unter R¹ genannten Reste können gegebenenfalls durch -SH, -SCH₃, -COOH, -CONH₂, -CHO, Guanidyl, -OH, -NR'R" oder -SS-CH₂-C(H)NH₂-CO₂H substituiert sein, wobei R' und R" die oben genannte Bedeutung haben.

R¹ ist bevorzugt Vinyl oder ein verzweigtes oder geradkettiges, gegebenenfalls substituiertes C₁-C₈-Alkyl, vorzugsweise Vinyl oder ein verzweigtes oder geradkettiges, gegebenenfalls substituiertes C₁-C₄-Alkyl wie Methyl, Ethyl, *n*-Propyl, *i*-Propyl, *n*-Butyl, *sek*-Butyl und *tert*-Butyl.

R¹ ist ganz besonders bevorzugt Vinyl, *n*-Propyl oder 2-(Methylthio)ethyl, insbesondere 2-(Methylthio)ethyl.

R² ist bevorzugt -OR"', worin R"' ein Wasserstoffatom oder eine verzweigte oder geradkettige C₁-C₄-Alkylgruppe bedeutet.

R³ ist unabhängig voneinander vorzugsweise ein Wasserstoffatom oder eine verzweigte oder geradkettige, gegebenenfalls substituierte C₁-C₄-Alkylgruppe, insbesondere *i*-Propyl oder *i*-Butyl, oder die beiden Reste R³ sind zusammen C₂-C₄-Alkandiyl und bilden gemeinsam einen Ring, oder die beiden Reste R³ und R"' sind zusammen Teil einer C₃-C₆-Alkantriylgruppe der allgemeinen Formel -R₃(CH-)R"'- und bilden gemeinsam eine bicyclische Gruppe.

R⁴ ist vorzugsweise Methyl oder Ethyl, insbesondere Methyl.

Bei dem erfindungsgemäßen Verfahren wird zunächst ein nicht cyclisches Dithioacetal gebildet (Schritt a)).

Dann wird das in a) erhaltene Dithioacetal durch eine Umpolungsreaktion mit einem carbonylhaltigen Elektrophil wie beispielsweise Kohlendioxid, Phosgen, Chlorameisensäureester, Orthoameisensäureester oder Dialkylcarbonat (Schritt b)) in Gegenwart einer Base zu einer α,α-(Dithio)carbonsäure oder deren Derivaten umgesetzt. Bevorzugt wird als Elektrophil Kohlendioxid verwendet, welches entweder als Gas, Feststoff (z.B. Trockeneis) oder Flüssigkeit (superkritisch oder als Lösungsmittel) eingesetzt werden kann.

Für die in b) zuerst erfolgende Deprotonierung sind Basen, deren korrespondierende Säure einen pKₐ-Wert > 20 aufweist, besonders geeignet. Bevorzugte Basen sind hierbei Alkyllithiumverbindungen, insbesondere *n*-Butyllithium und *tert*-Butyllithium, sowie Metallamide und -hydride, insbesondere Natriumamid und Natriumhydrid, außerdem Metallhydroxide oder -carbonate allein oder in Kombination mit chelatisierenden Liganden wie z.B. Kronenether, bevorzugt Kaliumhydroxid oder Kaliumcarbonat mit 18-Krone-6.

Die Deprotonierung in b) mit den vorgenannten Basen wird bevorzugt in aprotischen, organischen Lösungsmitteln oder in flüssigem überkritischen Kohlendioxid oder in flüssigem Ammoniak durchgeführt. Besonders bevorzugt sind dabei Ether wie Tetrahydrofuran, Alkylsulfoxide wie DMSO, aromatische Verbindungen wie Toluol, aliphatische und cyclische Alkane wie *n*-Hexan und Cyclohexan, sowie Alkylamide wie DMF.

Eine Temperatur von -80°C bis 100°C, bevorzugt von -30°C bis 25°C, ist für die Reaktion vorteilhaft.

Die Hydrolyse erfolgt bevorzugt in wässrigen Lösungen im pH-Bereich von 1-14, bevorzugt im Bereich von pH 1-4 oder pH 9-13.

Im Allgemeinen findet die Hydrolyse bei Temperaturen von - 20°C bis 100°C, vorzugsweise von 0 bis 30°C, statt.

Danach erfolgt in Schritt c) die sauer-katalysierte Solvolyse der in b) gebildeten α,α-(Dithio)-carbonsäure oder derer Derivate unter Freisetzung von Thiolen oder deren Salze, wobei das Produkt, jeweils abhängig vom verwendeten Lösungsmittel, beispielsweise die freie α-Ketosäure sowie deren Salze, ein α-Ketoester, ein α-Ketoamid, ein Ketalderivat der α-Ketosäure oder ein Ketalderivat des α-Ketoesters sein kann (siehe auch Schema).

Unter Solvolyse ist die Reaktion der α,α-(Dithio)-carbonsäure mit dem Lösungsmittel, z. B. die Hydrolyse (mit Wasser) zu verstehen.

Die Solvolyse erfolgt im Allgemeinen bei Temperaturen von 20°C - 200°C, bevorzugt bei 50-150°C, unter saurer Katalyse. Bevorzugte Katalysatoren sind beispielsweise *para-*Toluolsulfonsäure, CF₃SO₃H, sowie Mineralsäuren wie HCl oder H₂SO₄ oder starke organische Säuren, die nicht nucleophil sind.

Die gebildeten Thiole der Formel R⁴SH können durch ein Vakuum, durch Einleiten von Inertgas oder durch ein anderes geeignetes Trennverfahren wie zum Beispiel Phasentrennung, Kristallisation, Komplexbildung oder Fällung aus dem Reaktionsgemisch entfernt und recycelt werden und damit anschließend wieder in der Umpolungsreaktion verwendet werden.

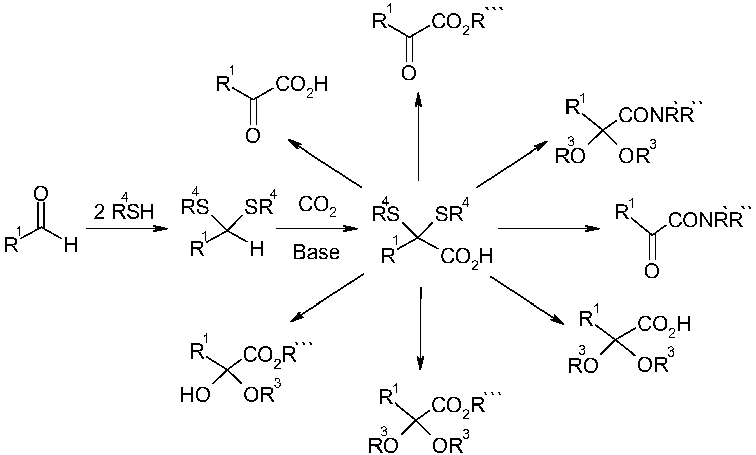

Gemäß einer Ausführungsform wird bei der säurekatalysierten Solvolyse in c) Wasser als Lösungsmittel verwendet, wobei sich die die freie α-Ketosäure der Formel (I) bildet, worin R² = OR"' ist, R"' ein Wasserstoffatom ist und R¹ die oben genannte Bedeutung hat.

Gemäß einer alternativen Ausführungsform wird bei der säurekatalysierten Solvolyse in c) ein Alkohol R"'OH, worin R"' eine verzweigte oder geradkettige C₁-C₈-Alkyl, C₅-C₈-Cycloalkyl-, Allyl- oder Benzylgruppe bedeutet und Alkyl gegebenenfalls substituiert ist, als Lösungsmittel verwendet, wobei sich der α-Ketoester der Formel (I) bildet, worin R² = OR"' und R"' die hier zuvor und R¹ die oben genannte Bedeutung haben.

Gemäß einer weiteren alternativen Ausführungsform wird bei der säurekatalysierten Solvolyse in c) ein Diol HO-R³-OH, wobei R³ eine verzweigte oder geradkettige, gegebenenfalls substituierte C₂-C₈-Alkandiylgruppe ist, als Lösungsmittel verwendet, wobei sich ein cyclisches Ketal der Formel (II) bildet, worin R² = OR"' mit R"' = H ist, R³ die hier zuvor und R¹ die oben genannte Bedeutung haben.

Gemäß einer weiteren alternativen Ausführungsform wird bei der säurekatalysierten Solvolyse in c) ein Triol der Formel HOR(CHOH)R"'OH, worin die Reste R³ und R''' zusammen Teil einer verzweigten oder geradkettigen, gegebenenfalls substituierten C₃-C₈-Alkantriylgruppe der allgemeinen Formel - R³ (CH-)R"'- sind, als Lösungsmittel verwendet, wobei sich ein bicyclischer Ketalester der Formel (II) bildet, worin R²= OR"' ist, R''' und R³ die hier zuvor und R¹ die oben genannte Bedeutung haben.

Gemäß einer weiteren alternativen Ausführungsform wird bei der säurekatalysierten Solvolyse in c) ein Amin HNR'R" , worin R' und R'' gleich oder verschieden sein können und ein Wasserstoffatom oder eine verzweigte oder geradkettige C₁-C₆-Alkylgruppe bedeuten, wobei R' und R" nicht gleichzeitig ein Wasserstoffatom bedeuten können, als Lösungsmittel verwendet. Dabei bildet sich ein Amid der Formel (I), worin R² = NR'R" ist und R' und R" die hier genannte Bedeutung haben.

Bei dem erfindungsgemäßen Verfahren zur Herstellung von α-Ketosäuren sowie deren Derivaten fungiert das in a) gebildete Dithioacetal auch als Schutzgruppe, die nach der Addition von Kohlendioxid (b)) in Gegenwart von einer Säure durch Solvolyse (c)) unter Vakuum oder durch Einleiten eines inerten Gases wie z.B. Stickstoff wieder entfernt werden kann. Von Vorteil ist, dass das dabei freigesetzte Thiol dann recycelt und durch einen Kreislaufprozess wieder eingesetzt werden kann.

Im Falle von α-Ketomethionin kann es vorteilhaft sein, das Dithioacetal in a) entweder durch Umsetzung von 3-(Methylthio)propanal (= 3-Methyl-mercaptopropionaldehyd (MMP)) mit Methylthiol (MeSH) oder direkt aus Acrolein durch die dreifache Addition von Methylthiol herzustellen.

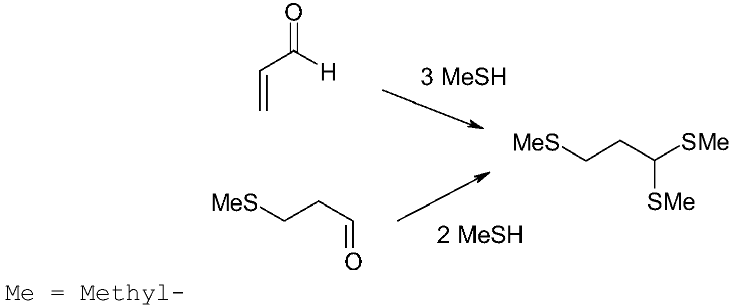

Im Vergleich zum Stand der Technik erlaubt das erfindungsgemäße Verfahren die Herstellung von α-Ketosäuren bzw. deren Derivaten in höherer Ausbeute, was einen großen wirtschaftlichen Vorteil darstellt.

Darüber hinaus können bei dem erfindungsgemäßen Verfahren die bestehenden Anlagen zur Herstellung von 3-(Methylthio)propanal, d.h. die Anlagen zur Herstellung von Acrolein sowie Methylthiol, weiter verwendet werden, im Gegensatz zu dem in WO 06-72711 offenbarten Verfahren.

Das erfindungsgemäße Verfahren ist zudem umweltfreundlich, da vorzugsweise Kohlendioxid als C-1 Baustein verwendet wird und damit zur Klimaschonung (Kyoto Protokoll) beiträgt.

Im Gegensatz zu dem in WO 06-72711 beschriebenen Verfahren zur Darstellung von α-Ketomethionin ausgehend von Butadien, weist das erfindungsgemäße Verfahren eine hohe Flexibilität auf, da sowohl die α-Ketosäure selbst, wie auch alle Derivate wie z. B. Ester, Ketale usw. direkt hergestellt werden können.

### Beispiele

### Beispiel 1:

### Herstellung von 1,1,3-Tris(methylthio)propan (2) aus 3-(Methylthio)propanal (1)

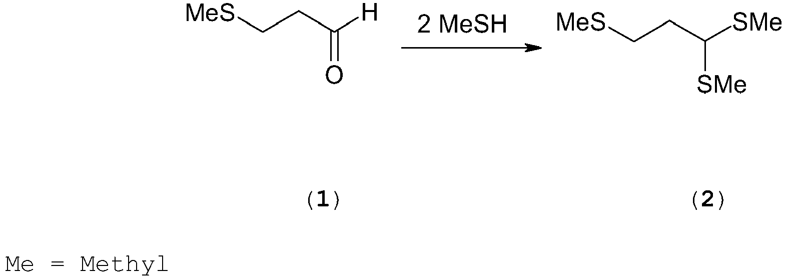

3-(Methylthio)propanal **(1)** (1.44 mol, 150 g) wurde mit HCl (g) bei 0°C gesättigt und anschließend direkt zu MeSH (6.25 mol, 300 g) bei 0°C über einen Zeitraum von 30 Minuten zugetropft. Das Reaktionsgemisch wurde auf 20°C erhitzt und 24 h lang bei 20°C gerührt. Nach Entfernen des überschüssigen MeSH unter Vakuum ergab sich laut GC-Analyse folgende Produktverteilung: 91% Thioacetal (**2**) und 8% 3-(Methylthio)propanal (**1**). Zur weiteren Aufreinigung wurde dieses Gemisch in Diethylether gelöst und mit einer 30%igen wässrigen Natriumpyrosulfit-(Na₂S₂O₅)-Lösung gewaschen. Nach Phasentrennung wurde die organische Phase über MgSO₄ getrocknet. Nach Entfernen des Lösungsmittels erhielt man das Thioacetal **(2)** als leicht gelb gefärbtes Öl (168 g, Ausbeute = 64%, GC-Reinheit = 98%).

¹H-NMR von 1,1,3-Tris(methylthio)propan **(2)** (500 MHz, CDCl₃): **δ** = 2.01-2.05 (m, 2H, CH₂), 2.11 (s, 9H, 3 x SCH₃), 2.71 (t, ³*J* = 7.3 Hz, 2H, CH₂), 3.83 (t, ³*J* = 7.3 Hz, 1H, CH)

¹³C-NMR von 1,1,3-Tris(methylthio)propan **(2)** (125.8 MHz, CDCl₃): δ = 12.6 (2 x SCH₃), 15.5 (SCH₃), 32.0 (CH₂), 33.9 (CH₂), 53.0 (C(SCH₃)₂)

### Beispiel 2:

### Herstellung von 1,1,3-Tris(methylthio)propan (2) aus Acrolein (3)

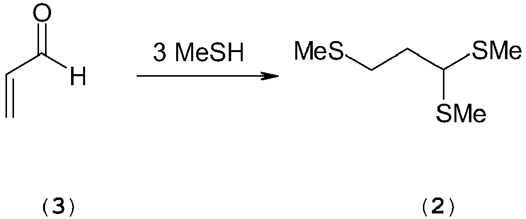

Zu MeSH (358 mmol, 17.2 g), der mit HCl-Gas bei 0°C gesättigt wurde, wurde Acrolein **(3)** (89 mmol, 5.0 g) tropfenweise über einen Zeitraum von 15 Minuten bei -78°C unter Rühren zugegeben. Diese Mischung wurde langsam auf 20°C erwärmt und anschließend 24 h lang bei 20°C weitergerührt. Nach Entfernen des überschüssigen MeSH unter Vakuum wurde dieses Gemisch in Diethylether gelöst und mit einer 30%igen wässrigen Natriumpyrosulfit-Lösung gewaschen. Nach Phasentrennung wurde die organische Phase über MgSO₄ getrocknet. Das reine Thioacetal **(2)** wurde nach Destillation (88°C bei 1.2 mbar) als leicht gelb gefärbtes Öl erhalten (11.4 g, Ausbeute = 70%, GC-Reinheit = 98%). Die NMR-Charakterisierung ergab die gleichen Daten wie im Beispiel 1.

### Beispiel 3:

### Darstellung von 2,2,4-Tris(methylthio)butansäure (4) durch Umpolungsreaktion von 1,1,3-Tris(methylthio)propan (2) mit Trockeneis (CO₂)

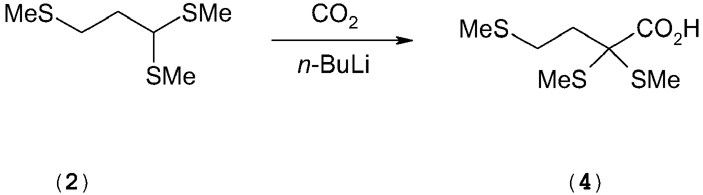

In einem Dreihalskolben wurde unter Schutzgasatmosphäre 1,1,3-Tris(methylthio)propan **(2)** (3.65 g, 20 mmol) in 50 ml abs. THF gelöst. Anschließend wurde bei -20°C eine Butyllithium Lösung in *n*-Hexan (14 ml, 1.6 M) unter Rühren langsam zugetropft. Dabei verfärbte sich die Lösung strahlend gelb. Nachdem bei dieser Temperatur 2 h lang weitergerührt wurde, wurde bei -70°C wasserfreies Trockeneis (CO₂, 10 g), welches zuvor in abs. THF gewaschen wurde, portionsweise zugegeben. Die Reaktionslösung wurde langsam auf 20°C aufgetaut und eine 10%ige wässerige KOH Lösung (80 ml) zugegeben. Nach Phasentrennung wurde die organische Phase mit 10%iger wässeriger KOH Lösung (2 x 50 ml) gewaschen. Die vereinten KOH-Phasen wurden mit Diethylether (3 x 30 ml) gewaschen und dann vorsichtig unter Kühlung mit konz. HCl auf pH 1 gestellt. Das Produkt wurde mit Diethylether (3 x 50 ml) extrahiert. Die vereinten Ether-Phasen wurden anschließend über Na₂SO₄ getrocknet und nach Filtration am Rotationsverdampfer eingeengt. Man erhielt 2,2,4-Tris(methylthio)butansäure **(4)** als ein gelbliches Öl (4.3 g, Ausbeute = 95%), welches langsam beim Stehen lassen kristallisiert.

¹H-NMR von 2,2,4-Tris(methylthio)butansäure **(4)**(500 MHz, CDCl₃): **δ** = 2.12 (s, 6H, 2 x SCH₃), 2.14 (s, 3H, SCH₃), 2.22-2.26 (m, 2H, CH₂), 2.67-2.70 (m, 2H, CH₂)

¹³C-NMR von 2,2,4-Tris(methylthio)butansäure **(4)** (125.8 MHz, CDCl₃): **δ** = 12.5 (2 x SCH₃), 15.6 (C-5), 29.7 (C-3), 34.6 (C-4), 63.5 (C-2), 175.3 (CO₂H)

### Beispiel 4:

### Darstellung von Calcium 2,2,4-Tris(methylthio)-butanoat (5) aus der entsprechenden α,α-(Dialkylthio)carbon-säuren (4)

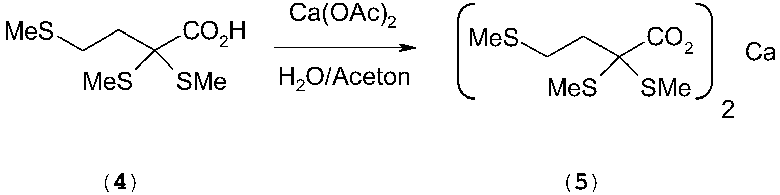

16.6 g 2,2,4-Tris(methylthio)butansäure **(4)** (73.3 mmol) wurden in einer Mischung aus 140 ml H₂O und 35 ml Aceton gelöst. Anschließend wurde eine Lösung aus 6.2 g Calciumacetat (93%ig) und 22 ml H₂O langsam bei 20°C unter starkem Rühren zugetropft. Nach kurzer Zeit fiel ein weißer Niederschlag aus, der nach 30 Minuten abfiltriert wurde. Der erhaltene weiße Feststoff wurde zweimal mit je 200 ml H₂O, dann mit je 200 ml Aceton und 200 ml Diethylether gewaschen und im Trockenschrank getrocknet. Ingesamt konnten 17.7 g an Calcium 2,2,4-Tris(methylthio)butanoat **(5)** (M = 490.8 g/mol, Ausbeute = 98%) isoliert werden.

¹H-NMR von Calcium 2,2,4-Tris(methylthio)butanoat **(5)**(500 MHz, DMSO-D₆): **δ** = 1.93 (s, 6H, 2 SCH₃), 1.95-1.99 (m, 2H, CH₂), 2.05 (s, 3H, SCH₃), 2.52-2.55 (m, 2H, CH₂)

### Beispiel 5:

### Darstellung von 2,2,4-Tris(methylthio)butansäure (4) durch Umpolungsreaktion von 1,1,3-Tris(methylthio)-propan (2) mit gasförmigen CO₂

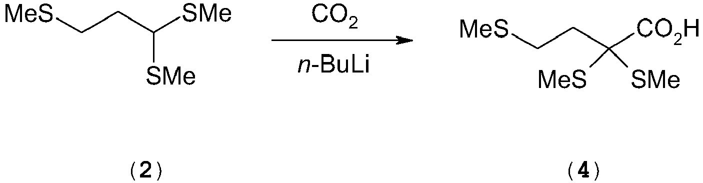

In einem Dreihalskolben wurde unter Schutzgasatmosphäre 1,1,3-Tris(methylthio)propan **(2)** (3.65 g, 20 mmol) in 50 ml abs. THF gelöst. Anschließend wurde bei -20°C eine Butyllithium Lösung in *n*-Hexan (14 ml, 1.6 M) unter Rühren langsam zugetropft. Dabei verfärbte sich die Lösung strahlend gelb. Nachdem bei dieser Temperatur 2 h lang weitergerührt wurde, wurde bei -70°C über eine Fritte gasförmiges, trockenes CO₂ über einen Zeitraum von 30 Minuten durchgeleitet. Die Reaktionslösung wurde langsam aufgetaut bis eine Temperatur von 20°C erreicht wurde, bei welcher eine 10%ige wässerige KOH Lösung (80 ml) zugegeben wurde.

Nach Phasentrennung wurde die organische Phase mit 10%iger wässeriger KOH Lösung (2 x 50 ml) gewaschen. Die vereinten KOH-Phasen wurden mit Diethylether (3 x 30 ml) gewaschen und dann vorsichtig unter Kühlung mit konz. HCl auf pH 1 gestellt. Das Produkt wurde mit Diethylether (3 x 50 ml) extrahiert. Die vereinten Ether-Phasen wurden anschließend über Na₂SO₄ getrocknet und nach Filtration am Rotationsverdampfer eingeengt. Man erhielt 2,2,4-Tris(methylthio)butansäure **(4)** als ein gelbliches Öl (4.1 g, Ausbeute = 90%), welches langsam beim Stehen lassen kristallisierte. Die NMR-Charakterisierung ergab die gleichen Daten wie im Beispiel 3.

### Beispiel 6:

### Herstellung des Ketomethionin-isopropylesters (6) durch Entfernung des Thiols aus 2,2,4-Tris(methylthio)butansäure (4) in Gegenwart von iso-Propanol

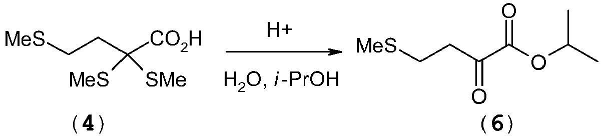

0.1 mol 2,2,4-Tris(methylthio)butansäure **(4)** (22.6 g) wurden in einer Mischung aus 200 ml Toluol und 200 ml *iso*-Propanol gelöst. Anschließend wurden 2.0 eq. H₂O (3.6 ml) und eine Spatelpitze p-Toluolsulfonsäure Monohydrat zugegeben. Die gesamte Mischung wurde dann auf Siedetemperatur erhitzt und 3 h lang unter Rückfluss gerührt. Nach Abkühlen wurden 150 ml Wasser zugegeben und dreimal mit je 100 ml Diethylether extrahiert. Die vereinten Etherphasen wurden anschließend mit einer verdünnten Natriumhydrogencarbonatlösung neutral gewaschen und über Magnesiumsulfat getrocknet. Nach Filtration wurde das gesamte Lösungsmittel mit einem Rotationsverdampfer abgezogen. Man erhielt den Ketomethionin-isopropylester **(6)** (14.5 g, Ausbeute = 76%) als leicht gelbliches Öl.

¹H-NMR von Ketomethionin-isopropylester **(6)(500** MHz, CDCl₃): **δ** = 1.35 (d, ³*J* = 6,3 Hz, 6H, 2 CH₃), 2.14 (s, 3H, SCH₃), 2.79 (t, ³*J* = 7,2 Hz, 2H, CH₂), 3.15 (t, ³*J =* 7,2 Hz, 2H, CH₂), 5.15 (quint, ³*J* = 6,3 Hz, 1H, CH)

Elementaranalyse für C₈H₁₄O₃S **(6)** (M = 190,26 g/mol) : C 50.50; H 7.43; S 16.85 Gefunden: C 50.66; H 7.57; S 16.52

### Beispiel 7:

### Direkte Herstellung von Ketomethioninethylenglykol-ketal (7) durch Entfernung des Thiols aus der 2,2,4-Tris(methylthio)butansäure (4) in Gegenwart von Ethylenglykol

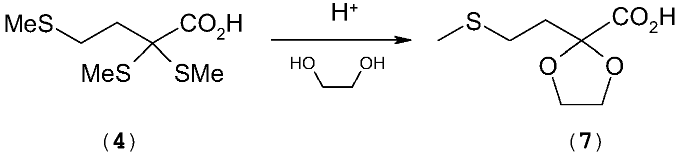

0.1 mol 2,2,4-Tris(methylthio)butansäure (4) (22.6 g) wurden in 200 ml Ethylenglykol (1,2-Ethandiol) gelöst. Anschließend wurden 1.1 eq. H₂O (1.8 ml) und eine Spatelpitze p-Toluolsulfonsäure Monohydrat zugegeben. Die gesamte Mischung wurde dann auf 50°C erwärmt und ein konstanter Stickstoffstrom über eine Fritte durch die Lösung geleitet. Nach 4 h wurde das Reaktionsgemisch in 300 ml Wasser gegeben und dreimal mit je 100 ml Diethylether extrahiert. Die vereinten Etherphasen wurden über MgSO₄ getrocknet. Nach Filtration wurde der Ether mit einem Rotationsverdampfer abgezogen. Zu dem als Ketal geschützten Ester wurden anschließend 100 ml Methanol und 100 ml 2 M NaOH-Lauge gegeben und 2 h lang bei 20°C gerührt. Danach wurde die Lösung mit konz. HCl auf pH = 1 angesäuert und dreimal mit je 100 ml Diethylether extrahiert. Die vereinten Etherphasen wurden anschließend über MgSO₄ getrocknet. Nach Filtration wurde der Ether abgezogen und das Rohprodukt aus einer Mischung von Methylenchlorid und *n*-Hexan auskristallisiert. Man erhielt 13.0 g eines weißen kristallinen Feststoffs **(7).** (Ausbeute = 68%, M = 192.23 g/mol, Schmelzpunkt: 74°C (kristallisiert aus Methylenchlorid/n-Hexan).

¹H-NMR von 2-(2-(Methylthio)ethyl)-1,3-dioxolan-2-carbonsäure **(7)** (500 MHz, CDCl₃): **δ =** 2.11 (s, 3H, SCH₃), 2.24-2.28 (m, 2H, CH₂), 2.58-2.61 (m, 2H, CH₂), 4.07-4.14 (m, 4H, OCH₂CH₂O)

¹³C-NMR von 2-(2-(Methylthio)ethyl)-1,3-dioxolan-2-carbonsäure **(7)** (125.8 MHz, CDCl₃): **δ** = 15.5 (SCH₃), 27.1 (CH₂), 34.9 (CH₂), 66. 1 (2 OCH₂), 105. 9 (C), 174.1 (COO)

Elementaranalyse für C₇H₁₂O₄S **(7)** (M = 192,24 g/mol) : C 43.74; H 6.29; S 16.68 Gefunden: C 43.80; H 6.25; S 16.61

### Beispiel 8:

### Direkte Herstellung von Ketomethioninketalester (8a)/(8b) durch Entfernung des Thiols aus der α,α-(Dialkylthio)-carbonsäure (4) in der Gegenwart von Glycerin

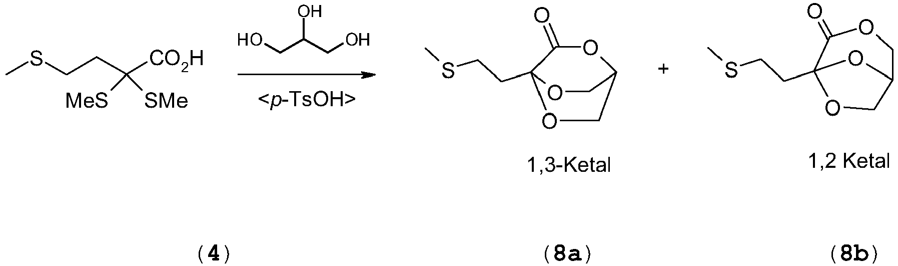

0.1 mol 2,2,4-Tris(methylthio)butansäure **(4)** (22.6 g) wurden in 100 ml Glycerin (1,2,3-Propantriol) gelöst. Anschließend wurden 1.1 eq. H₂O (1.8 ml) und eine Spatelpitze p-Toluolsulfonsäure Monohydrat zugegeben. Die gesamte Mischung wurde dann auf 70°C erwärmt und ein Vakuum von 750 mbar angelegt. Nach 5.5 h wurde das Reaktionsgemisch in 300 ml Wasser gegeben und dreimal mit je 100 ml Diethylether extrahiert. Die vereinten Etherphasen wurden über MgSO₄ getrocknet. Nach Filtration wurde der Ether am Rotationsverdampfer entfernt und das ölige Rohprodukt (Verhältnis (**8a**)**:**(**8b**)=70:30) aus einer Mischung von Methylenchlorid/n-Hexan kristallisiert. Das Hauptprodukt (**8a**) kristallisierte in Form farbloser Nadeln aus (9.2 g, Ausbeute = 45%, M = 204.25 g/mol, Schmelzpunkt = 39.5°C (umkristallisiert aus Methylenchlorid/n-Hexan)).

¹H-NMR von 4-(2-(Methylthio)ethyl)-2,5,8-trioxabicyclo[2.2.2]-octan-3-on (**8a**) (500 MHz, CDCl₃): **δ** = 2.13 (s, 3H, SCH₃), 2.17-2.20 (m, 2H, CH₂), 2.65-2.68 (m, 2H, CH₂), 4.12-4.13 (m, 4H, 2 CH₂), 4.76 (s, 1H, CH)

¹³C-NMR von 4-(2-(Methylthio)ethyl)-2,5,8-trioxabicyclo[2.2.2]-octan-3-on (**8a**) (125.8 MHz, CDCl₃): **δ** = 15.4 (SCH₃), 26.9 (CH₂), 33.2 (CH₂), 66.5 (2 OCH₂), 70.9 (CH), 92.9 (C), 166.2 (COO)

Elementaranalyse für C₈H₁₂O₄S (**8a**) (M = 204,25 g/mol) : C 47.04; H 5.92; S 15.70 Gefunden: C 47.21; H 5.93; S 15.69

### Beispiel 9:

### Herstellung von 1,1-Bis(methylthio)butan (10)

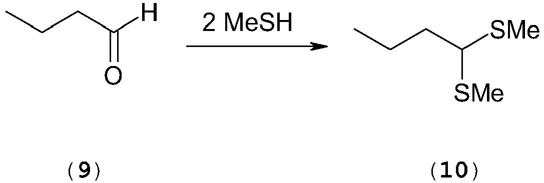

10.0 g Butanal **(9)** (139 mmol) wurde mit HCl (g) bei 0°C gesättigt und anschließend direkt zu MeSH (624 mmol, 30.0 g) bei 0°C über einen Zeitraum von 25 Minuten zugetropft. Das Reaktionsgemisch wurde auf 20°C erwärmt und 17 h lang bei 20°C gerührt. Zur weiteren Aufreinigung wurde dieses Gemisch in Diethylether gelöst und mit einer 30%igen wässerigen Natriumpyrosulfit-Lösung
gewaschen. Nach Phasentrennung wurde die organische Phase über Na₂SO₄ getrocknet. Nach Entfernen des Lösungsmittels erhielt man das Thioacetal **(10)** als klares, farbloses Öl (17.3 g, Ausbeute = 83%; GC-Reinheit 98%). Die NMR-Daten stimmen mit denen aus der Literatur überein.

### Beispiel 10:

### Darstellung von 1,1-Bis(methylthio)pentansäure (11) durch Umpolungreaktion von 1,1-Bis(methylthio)butan (10) mit Trockeneis (CO₂)

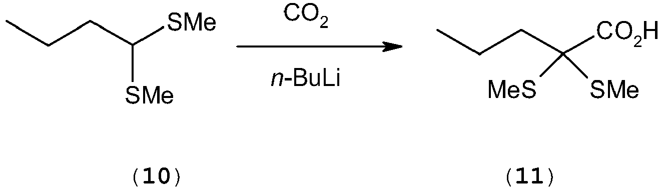

10 mmol 1,1-Bis(methylthio)butan **(10)** (1.26 g) wurden unter Stickstoff-Atmosphäre in 25 ml getrocknetem THF vorgelegt und auf -20°C abgekühlt. Anschließend wurden 11 mmol 1.6 M n-BuLi-Lsg. in *n*-Hexan (7.0 mL) bei -20°C über einen Zeitraum von 5 Minuten zugetropft und 2 h lang gerührt. Die klare, leicht gelbe Reaktionslösung wurde zu 15 g Trockeneis bei -78°C zugetropft und 36 h lang gerührt, wobei die Temperatur über Nacht auf 20°C anstieg. Anschließend wurde das Reaktionsgemisch mit 40 ml 10%iger KOH-Lsg. versetzt und die Phasen getrennt. Die org. Phase wurde zweimal mit je 25 ml 10%iger KOH-Lsg. und die vereinigten KOH-Phasen dreimal mit je 25 ml Methyl-*tert*-butylether (MTBE) gewaschen. Die KOH-Phase wurde dann mit konz. HCl (aq) sauer gestellt (pH 1) und viermal mit je 25 ml MTBE extrahiert. Die vereinigten organischen Phasen wurden über MgSO₄ getrocknet und mit einem Rotationsverdampfer eingeengt. Man erhielt die 2,2-(Dimethylthio)pentansäure **(11)** als weißen Feststoff (1.0 g, Ausbeute = 51.5%, Schmelzpunkt = 85°C (umkristallisiert aus einer Mischung aus Chloroform, Methanol und Essigsäure)).

¹H-NMR von 2,2-(Dimethylthio)pentansäure **(11)** (500 MHz, CDCl₃): **δ** = 0.96 (t, ³*J* = 7.4 Hz, 3H, C⁵H₃), 1.50-1.60 (m, 2H, C⁴H₂), 1.88-1.94 (m, 2H, C³H₂), 2.10 (s, 6H, 2 x SCH₃), 11.5 (bs, 1H, C¹OOH)

¹³C-NMR von 2,2-(Dimethylthio)pentansäure (11) (125.8 MHz, CDCl₃): **δ** = 12.5 (2 x SCH₃), 14.0 (C-5), 15.1 (C-4), 36.9 (C-3), 64.6 (C-2), 176.3 (C-1)

### Beispiel 11:

### Herstellung von 2-Oxovaleriansäureethylester (12) durch Entfernung des Thiols aus 2,2-(Dimethylthio)pentansäure (11) in Gegenwart von Wasser und Ethanol

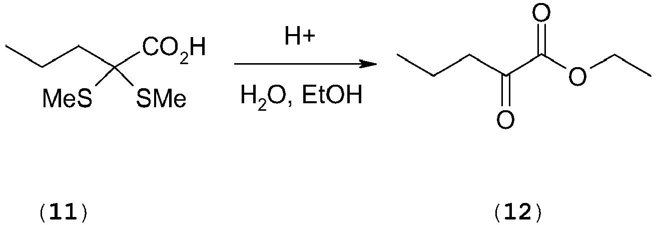

0.1 mol 2,2-(Dimethylthio)pentansäure **(11)** (19.4 g) wurden in 150 ml Toluol gelöst und mit 2.0 eq. H₂O (3.6 ml) und einer Spatelpitze p-Toluolsulfonsäure Monohydrat versetzt. Anschließend wurde auf Siedetemperatur erhitzt und ein Stickstoffstrom über eine Fritte durch die Lösung geleitet. Nach 3 h unter Rückfluss wurde die Reaktionsmischung auf 80°C abgekühlt und mit 200 ml Ethanol und einer weiteren Spatelspitze *p*-Toluolsulfonsäure Monohydrat versetzt. Nach weiteren 3 h unter Rückfluss wurde auf 20°C abgekühlt, 200 ml Wasser zugegeben und dreimal mit je 100 ml Diethylether extrahiert. Die vereinten Etherphasen wurden anschließend mit einer verdünnten Natriumhydrogencarbonatlösung neutral gewaschen und über Magnesiumsulfat getrocknet. Nach Filtration wurde das gesamte Lösungsmittel mit einem Rotationsverdampfer abgezogen. Man erhielt den 2-Oxovaleriansäureethylester **(12)** (10.2 g, Ausbeute = 71%) als leicht gelbliches Öl.

¹H-NMR von 2-Oxovaleriansäureethylester **(12)**(500 MHz, CDCl₃): **δ** = 0.97 (t, ³*J* = 7.3 Hz, 3H, C⁵H₃), 1.37 (t, ³*J* = 7.3 Hz, 3H, OCH₂CH₃), 1.67 (sext, ³*J* = 7.3 Hz, 2H, C⁴H₂) , 2.81 (t, ³*J* = 7.3 Hz, 2H, C³H₃), 4.32 (q, ³*J* = 7.3 Hz, 2H, OCH₂CH₃)

¹³C-NMR von 2-Oxovaleriansäureethylester **(12)** (125.8 MHz, CDCl₃): δ = 13.5 (OCH₂CH₃), 14.0 (C-5), 16.6 (C-4), 41.2 (C-3), 62.3 (OCH₂CH₃), 161.4 (C-1), 194.7 (C-2)

### Beispiel 12:

### Herstellung von 2-Oxovaleriansäure (13) durch Entfernung des Thiols aus 2,2-(Dimethylthio)pentansäure (11) in Gegenwart von Wasser

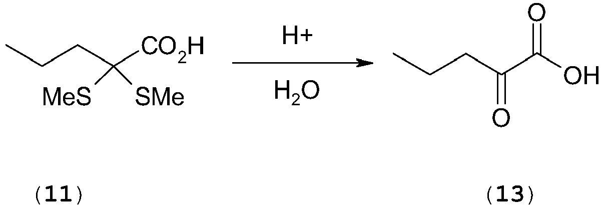

10.0 mmol 2,2-(Dimethylthio)pentansäure **(11)** (1.94 g) wurden in 30 ml rauchender Salzsäure (37%ig) suspendiert und mit einer Spatelspitze *p*-Toluolsulfonsäure-Monohydrat versetzt. Anschließend wurde auf Siedetemperatur erhitzt und 5 h lang gerührt. Dabei wurde ein Vakuum von 750 mbar angelegt. Nach der Reaktion wurde mit 100 ml Wasser verdünnt und dreimal mit je 80 ml Diethylether extrahiert. Die vereinigten org. Phasen wurden dreimal mit je 100 ml

Natriumhydrogencarbonatlösung gewaschen. Die vereinten wässrigen Phasen wurden anschließend mit konzentrierter Salzsäure auf pH = 1 angesäuert und dreimal mit je 100 ml Diethylether extrahiert. Die vereinten Etherphasen wurden über MgSO₄ getrocknet, filtriert und das Filtrat bei Raumtemperatur im Vakuum zur Trockne eingeengt. Man erhielt 2-Oxovaleriansäure **(13)** als farbloses Öl (0.72 g, Ausbeute = 62%).

¹H-NMR von 2-Oxovaleriansäure **(13)(500** MHz, DMSO-D6): **δ** = 0.88 (t, ³*J* = 7.4 Hz, 3H, C⁵H₃), 1.53 (sext, ³*J* = 7.4 Hz, 2H, C⁴H₂), 1.96 (t, ³*J* = 7.4 Hz, 2H, C³H₃), 13.5 (bs, 1H, COOH)

¹³C-NMR von 2-Oxovaleriansäure **(13)** (125.8 MHz, DMSO-D6): **δ** = 13.64 (C-5), 16.37 (C-4), 40.56 (C-3), 163.28 (C-1), 196.93 (C-2)

## Patentansprüche

1. Verfahren zur Herstellung von α-Ketosäuren sowie deren Derivaten der allgemeinen Formel **(I)** oder **(II),** 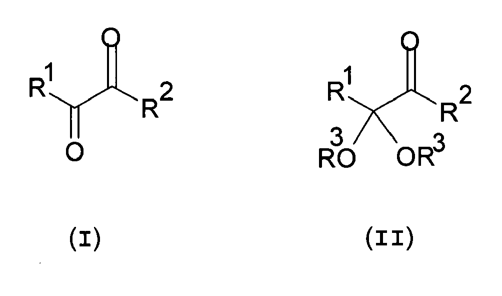 worin
R¹ eine verzweigte oder geradkettige C₁-C₁₈-Alkyl-, C₅-C₈-Cycloalkyl-, Vinyl-, Allyl-, C₆-C₁₀-Aryl-C₁-C₄-alkylen oder C₄-C₉-Heteroaryl-C₁-C₄-alkylen-Gruppe bedeutet, wobei R¹ gegebenenfalls substituiert ist,
R² -OR''' oder -NR'R'' bedeutet, worin R' und R" gleich oder verschieden sind und ein Wasserstoffatom oder eine verzweigte oder geradkettige C₁-C₆-Alkylgruppe bedeuten, und R"' ein Wasserstoffatom, eine verzweigte oder geradkettige C₁-C₈-Alkyl-, C₅-C₈-Cycloalkyl-, Allyl- oder eine Benzylgruppe bedeutet, wobei Alkyl in R', R" und/oder R''' gegebenenfalls substituiert ist, oder R"' ein Alkali-, Erdalkali- oder ein ein- oder zweiwertiges Übergangsmetallion bedeutet,
R³ gleich oder verschieden ist und ein Wasserstoffatom, eine verzweigte oder geradkettige C₁-C₈-Alkyl-, Allyl-oder eine Benzylgruppe bedeutet, wobei Alkyl und/oder Benzyl gegebenenfalls substituiert ist, oder die beiden Reste R³ zusammen C₂-C₈-Alkandiyl sind und gemeinsam einen Ring bilden, oder die beiden Reste R³ und R''' zusammen Teil einer C₃-C₈-Alkantriyl-Gruppe der allgemeinen Formel -R³(CH-)R"'- sind und gemeinsam eine bicyclische Gruppe bilden,
wobei
a) ein Aldehyd R¹CHO mit gleichen oder verschiedenen Thiolen der Formel R⁴-S-H, worin R⁴ eine verzweigte oder geradkettige, gegebenenfalls substituierte C₁-C₆-Alkyl-, C₅-C₈-Cycloalkyl-, Allyl- oder Benzylgruppe ist, zum entsprechenden Dithioacetal umgesetzt wird,
b) das gebildete Dithioacetal mit einem carbonylhaltigen Elektrophil in Gegenwart einer starken Base und nach Hydrolyse zur α,α-(Dithio)carbonsäure oder deren Derivaten der Formel **(III)** 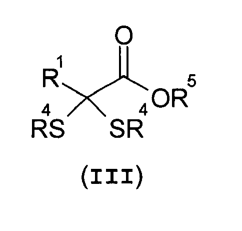 reagiert, worin R⁵ ein Wasserstoffatom oder eine verzweigte oder geradkettige C₁-C₆-Alkylgruppe bedeutet und die Reste R⁴ und R¹ die oben genannte Bedeutung haben, und
c) die α,α-(Dithio)carbonsäure oder deren Derivate der Formel **(III)** durch säurekatalysierte Solvolyse in Gegenwart von mindestens 1 Moläquivalent Wasser unter Freisetzung von Thiolen der Formel R⁴SH zur α-Ketosäure oder deren Derivaten der allgemeinen Formel **(I)** oder **(II)** umgesetzt wird.

2. Verfahren nach Anspruch 1 **dadurch gekennzeichnet, dass** die in b) zur Deprotonierung verwendete starke Base, gemessen an ihrer korrespondierenden Säure, einen pKₐ-Wert > 20 aufweist.

3. Verfahren nach Anspruch 1 oder 2, **dadurch gekennzeichnet, dass** die Deprotonierung in b) in aprotischen, organischen Lösungsmitteln, oder in flüssigem überkritischem Kohlendioxid oder in flüssigem Ammoniak durchgeführt wird.

4. Verfahren nach einem oder mehreren der Ansprüche 1-3, **dadurch gekennzeichnet, dass** der Verfahrensschritt b) bei einer Temperatur von -80°C bis 100°C erfolgt.

5. Verfahren nach einem oder mehreren der Ansprüche 1-4, **dadurch gekennzeichnet, dass** das in c) freigesetzte Thiol der Formel R⁴SH aus dem Reaktionsgemisch entfernt, anschließend recycelt und bei a) wieder eingesetzt wird.

6. Verfahren nach Anspruch 5, **dadurch gekennzeichnet, dass** das Thiol der Formel R⁴SH ein C₁-C₆-Alkylthiol ist, welches durch ein Vakuum oder durch das Durchleiten von Inertgas aus dem Reaktionsgemisch entfernt wird.

7. Verfahren nach einem oder mehreren der Ansprüche 1 bis 6, **dadurch gekennzeichnet, dass** in a) Methylthiol als Thiol eingesetzt wird.

8. Verfahren nach einem oder mehreren der Ansprüche 1-7, **dadurch gekennzeichnet, dass** die säurekatalysierte Solvolyse in c) mit Wasser als Lösungsmittel erfolgt.

9. Verfahren nach einem oder mehreren der Ansprüche 1-7, **dadurch gekennzeichnet, dass** die säurekatalysierte Solvolyse in c) mit einem Alkohol R'''OH als Lösungsmittel erfolgt, worin R''' eine verzweigte oder geradkettige C₁-C₈-Alkyl-, C₅-C₈-Cycloalkyl-, Allyl- oder eine Benzylgruppe bedeutet und Alkyl gegebenenfalls substituiert ist.

10. Verfahren nach einem oder mehreren der Ansprüche 1-7, **dadurch gekennzeichnet, dass** die säurekatalysierte Solvolyse in c) mit einem Diol HO-R³-OH als Lösungsmittel erfolgt, wobei R³ eine verzweigte oder geradkettige, gegebenenfalls substituierte C₂-C₈-Alkandiylgruppe ist.

11. Verfahren nach einem oder mehreren der Ansprüche 1-7, **dadurch gekennzeichnet, dass** die säurekatalysierte Solvolyse in c) mit einem Triol HOR³(CHOH)R'''OH als Lösungsmittel erfolgt, wobei die Reste R³ und R''' zusammen Teil einer verzweigten oder geradkettigen, gegebenenfalls substituierten C₃-C₈-Alkantriylgruppe der allgemeinen Formel -R³(CH-)R"'- sind.

12. Verfahren nach einem oder mehreren der Ansprüche 1-7, **dadurch gekennzeichnet, dass** die säurekatalysierte Solvolyse in c) mit einem Amin HNR'R'' als Lösungsmittel erfolgt, worin R' und R'' gleich oder verschieden sein können und ein Wasserstoffatom oder eine verzweigte oder geradkettige C₁-C₆-Alkylgruppe bedeuten, aber R' und R'' nicht gleichzeitig ein Wasserstoffatom bedeuten können.

13. Verfahren nach einem oder mehreren der Ansprüche 1-12, **dadurch gekennzeichnet, dass** in a) ein Aldehyd R¹CHO, worin R¹ Vinyl oder CH₃SCH₂CH₂- ist, verwendet wird.

14. Verfahren nach einem oder mehreren der Ansprüche 1-13, **dadurch gekennzeichnet, dass** die α-Ketosäure der allgemeinen Formel **(I)** für α-Ketomethionin sowie seine Derivate steht, wobei R¹ CH₃SCH₂CH₂ bedeutet und in Schritt a) ein Aldehyd R¹CHO, worin R¹ = CH₃SCH₂CH₂- ist, mit Methylthiol umgesetzt wird.

15. Verfahren nach einem oder mehreren der Ansprüche 1-13, **dadurch gekennzeichnet, dass** die α-Ketosäure der allgemeinen Formel **(I)** für α-Ketomethionin sowie seine Derivate steht, wobei R¹ Vinyl bedeutet und in Schritt a) die dreifache Addition von Methylthiol an Acrolein stattfindet.

## Claims

1. Process for preparing α-keto acids and derivatives thereof of the general formula (I) or (II) 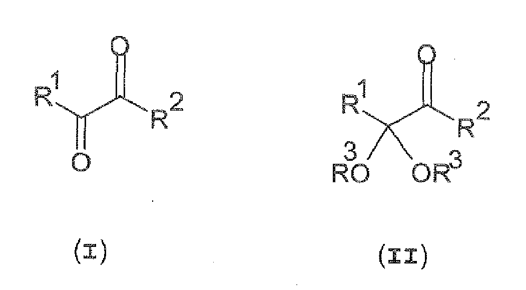 in which
R¹ is a branched or straight-chain C₁-C₁₈-alkyl, C₅-C₈-cycloalkyl, vinyl, allyl, C₆-C₁₀-aryl-C₁-C₄-alkylene or C₄-C₉-heteroaryl-C₁-C₄-alkylene group, where R¹ is optionally substituted,
R² is -OR''' or -NR'R'', in which R' and R" are the same or different and are each a hydrogen atom or a branched or straight-chain C₁-C₆-alkyl group, and R''' is a hydrogen atom, a branched or straight-chain C₁-C₈-alkyl, C₅-C₈-cycloalkyl, allyl or a benzyl group, where alkyl in R', R'' and/or R"' is optionally substituted, or R"' is an alkaline metal ion, alkaline earth metal ion or a mono- or divalent transition metal ion,
R³ is the same or different and is a hydrogen atom, a branched or straight-chain C₁-C₈-alkyl, allyl or a benzyl group, where alkyl and/or benzyl is optionally substituted, or the two R³ radicals together are C₂-C₈-alkanediyl and together form a ring, or the two R³ and R"' radicals together are part of a C₃-C₈-alkanetriyl group of the general formula -R³(CH-)R'" and together form a bicyclic group,
wherein
a) an aldehyde R¹CHO is reacted with identical or different thiols of the formula R⁴-S-H, in which R⁴ is a branched or straight-chain, optionally substituted C₁-C₆-alkyl, C₅-C₈-cycloalkyl, allyl or benzyl group to give the corresponding dithioacetal,
b) the dithioacetal formed reacts with a carbonyl-containing electrophile in the presence of a strong base and after hydrolysis to give the α,α-(dithio)carboxylic acid or derivatives thereof of the formula **(III)** 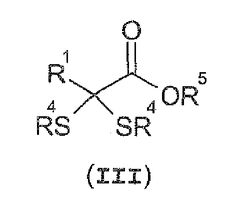 in which R⁵ is a hydrogen atom or a branched or straight-chain C₁-C₆-alkyl group and the R⁴ and R¹ radicals are each as defined above, and c) the **α,α-**(dithi**o**)carboxylic acid or derivatives thereof of the formula **(III)** are converted by acid-catalyzed solvolysis in the presence of at least 1 molar equivalent of water with release of thiols of the formula R⁴SH to give the α-keto acid or derivatives thereof of the general formula **(I)** or **(II).**

2. Process according to Claim 1, **characterized in that** the strong base used for deprotonation in b), measured on its corresponding acid, has a pKₐ value of > 20.

3. Process according to Claim 1 or 2, **characterized in that** the deprotonation in b) is performed in aprotic organic solvents or in liquid supercritical carbon dioxide or in liquid ammonia.

4. Process according to one or more of Claims 1-3, **characterized in that** process step b) is effected at a temperature of -80°C to 100°C.

5. Process according to one or more of Claims 1-4, **characterized in that** the thiol of the formula R⁴SH released in c) is removed from the reaction mixture, then recycled and reused in a).

6. Process according to Claim 5, **characterized in that** the thiol of the formula R⁴SH is a C₁-C₆-alkylthiol which is removed from the reaction mixture by a vacuum or by passing inert gas through.

7. Process according to one or more of Claims 1-6, **characterized in that** the thiol used in a) is methylthiol.

8. Process according to one or more of Claims 1-7, **characterized in that** the acid-catalyzed solvolysis in c) is effected with water as the solvent.

9. Process according to one or more of Claims 1-7, **characterized in that** the acid-catalyzed solvolysis in c) is effected with an alcohol R"'OH as the solvent, in which R''' is a branched or straight-chain C₁-C₈-alkyl, C₅-C₈-cycloalkyl, allyl or a benzyl group, and alkyl is optionally substituted.

10. Process according to one or more of Claims 1-7, **characterized in that** the acid-catalyzed solvolysis in c) is effected with a diol HO-R³-OH as the solvent, where R³ is a branched or straight-chain, optionally substituted C₂-C₈-alkanediyl group.

11. Process according to one or more of Claims 1-7, **characterized in that** the acid-catalyzed solvolysis in c) is effected with a triol HOR³(CHOH)R'"OH as the solvent where the R³ and R"' radicals together are part of a branched or straight-chain, optionally substituted C₃-C₈-alkanetriyl group of the general formula -R³(CH-)R'''.

12. Process according to one or more of Claims 1-7, **characterized in that** the acid-catalyzed solvolysis in c) is effected with an amine HNR'R'' as the solvent, in which R' and R" may be the same or different and are each a hydrogen atom or a branched or straight-chain C₁-C₆-alkyl group, but R' and R" cannot both be a hydrogen atom.

13. Process according to one or more of Claims 1-12, **characterized in that** an aldehyde R¹CHO in which R¹ is vinyl or CH₃SCH₂CH₂- is used in a).

14. Process according to one or more of Claims 1-13, **characterized in that** the α-keto acid of the general formula (I) is α-ketomethionine and derivatives thereof, where R¹ is CH₃SCH₂CH₂, and an aldehyde R¹CHO in which R¹ = CH₃SCH₂CH₂- is reacted with methylthiol in step a).

15. Process according to one or more of Claims 1-13, **characterized in that** the α-keto acid of the general formula (I) is α-ketomethionine and derivatives thereof, where R¹ is vinyl, and the triple addition of methylthiol to acrolein takes place in step a).

## Revendications

1. Procédé de fabrication d'α-cétoacides et de leurs dérivés de formule générale (I) ou (II) 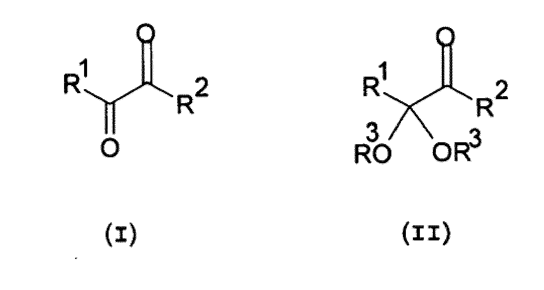 dans lesquelles
R¹ signifie un groupe alkyle en C₁-C₁₈, cycloalkyle en C₅-C₈, vinyle, allyle, aryle en C₆-C₁₀-alkylène en C₁-C₄ ou hétéroaryle en C₄-C₉-alkylène en C₁-C₄ ramifié ou linéaire, R¹ étant éventuellement substitué,
R² signifie -OR"' ou NR'R", R' et R" étant identiques ou différents et signifiant un atome d'hydrogène ou un groupe alkyle en C₁-C₆ ramifié ou linéaire, et R"' signifiant un atome d'hydrogène, un groupe alkyle en C₁-C₈, cycloalkyle en C₅-C₈, allyle ou benzyle ramifié ou linéaire, alkyle dans R', R'' et/ou R''' étant éventuellement substitué, ou R"' signifiant un ion de métal alcalin, de métal alcalino-terreux ou de métal de transition mono- ou bivalent,
les R³ sont identiques ou différents et signifient un atome d'hydrogène, un groupe alkyle en C₁-C₈ allyle ou benzyle ramifié ou linéaire, alkyle et/ou benzyle étant éventuellement substitués, ou les deux radicaux R³ représentent ensemble alcanediyle en C₂-C₈ et forment ensemble un cycle, ou les deux radicaux R³ et R"' font ensemble partie d'un groupe alcanetriyle en C₃-C₈ de formule générale -R³(CH-)R'''- et forment ensemble un groupe bicyclique,
selon lequel
a) un aldéhyde R¹CHO est mis en réaction avec des thiols identiques ou différents de formule R⁴-S-H, R⁴ étant un groupe alkyle en C₁-C₆, cycloalkyle en C₅-C₈, allyle ou benzyle ramifié ou linéaire, éventuellement substitué, pour former le dithioacétal correspondant,
b) le dithioacétal formé est mis en réaction avec un électrophile contenant un carbonyle en présence d'une base forte et par hydrolyse pour former l'acide α,α-(dithio)carboxylique ou ses dérivés de formule (III) 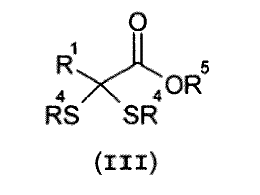 R⁵ signifiant un atome d'hydrogène ou un groupe alkyle en C₁-C₆ ramifié ou linéaire, et les radicaux R⁴ et R¹ ayant la signification indiquée précédemment, et
c) l'acide α,α-(dithio)carboxylique ou ses dérivés de formule (III) sont mis en réaction par solvolyse sous catalyse acide en présence d'au moins 1 équivalent molaire d'eau avec libération de thiols de formule R⁴SH pour former l'α-cétoacide ou ses dérivés de formule générale (I) ou (II).

2. Procédé selon la revendication 1, **caractérisé en ce que** la base forte utilisée en b) pour la déprotonation présente une valeur de pKₐ > 20, mesurée sur son acide correspondant.

3. Procédé selon la revendication 1 ou 2, **caractérisé en ce que** la déprotonation en b) est réalisée dans des solvants organiques aprotiques ou dans du dioxyde de carbone supercritique liquide ou dans de l'ammoniac liquide.

4. Procédé selon une ou plusieurs des revendications 1 à 3, **caractérisé en ce que** l'étape de procédé b) a lieu à une température de -80 °C à 100 °C.

5. Procédé selon une ou plusieurs des revendications 1 à 4, **caractérisé en ce que** le thiol de formule R⁴SH libéré en c) est éliminé du mélange réactionnel, puis recyclé et réutilisé en a).

6. Procédé selon la revendication 5, **caractérisé en ce que** le thiol de formule R⁴SH est un alkylthiol en C₁-C₆, qui est éliminé du mélange réactionnel par un vide ou par le passage d'un gaz inerte.

7. Procédé selon une ou plusieurs des revendications 1 à 6, **caractérisé en ce que** du méthylthiol est utilisé en a) en tant que thiol.

8. Procédé selon une ou plusieurs des revendications 1 à 7, **caractérisé en ce que** la solvolyse sous catalyse acide en c) a lieu avec de l'eau en tant que solvant.

9. Procédé selon une ou plusieurs des revendications 1 à 7, **caractérisé en ce que** la solvolyse sous catalyse acide en c) a lieu avec un alcool R'"OH en tant que solvant, R"' signifiant un groupe alkyle en C₁-C₈, cycloalkyle en C₅-C₈, allyle ou benzyle ramifié ou linéaire, et alkyle étant éventuellement substitué.

10. Procédé selon une ou plusieurs des revendications 1 à 7, **caractérisé en ce que** la solvolyse sous catalyse acide en c) a lieu avec un diol HO-R³-OH en tant que solvant, R³ étant un groupe alcanediyle en C₂-C₈ ramifié ou linéaire, éventuellement substitué.

11. Procédé selon une ou plusieurs des revendications 1 à 7, **caractérisé en ce que** la solvolyse sous catalyse acide en c) a lieu avec un triol HOR³(CHOH)R"'OH en tant que solvant, les radicaux R³ et R"' faisant ensemble partie d'un groupe alcanetriyle en C₃-C₈ ramifié ou linéaire, éventuellement substitué, de formule générale -R³(CH-)R"'.

12. Procédé selon une ou plusieurs des revendications 1 à 7, **caractérisé en ce que** la solvolyse sous catalyse acide en c) a lieu avec une amine HNR'R" en tant que solvant, R' et R'' pouvant être identiques ou différents et signifiant un atome d'hydrogène ou un groupe alkyle en C₁-C₆ ramifié ou linéaire, R' et R" ne pouvant toutefois pas signifier simultanément un atome d'hydrogène.

13. Procédé selon une ou plusieurs des revendications 1 à 12, **caractérisé en ce qu'**un aldéhyde R¹CHO est utilisé en a), R¹ étant vinyle ou CH₃SCH₂CH₂-.

14. Procédé selon une ou plusieurs des revendications 1 à 13, **caractérisé en ce que** l'α-cétoacide de formule générale (I) représente l'α-cétométhionine et ses dérivés, R¹ signifiant CH₃SCH₂CH₂ et un aldéhyde R¹CHO, avec R¹ = CH₃SCH₂CH₂-, étant mis en réaction avec du méthylthiol à l'étape a).

15. Procédé selon une ou plusieurs des revendications 1 à 13, **caractérisé en ce que** l'α-cétoacide de formule générale (I) représente l'α-cétométhionine et ses dérivés, R¹ signifiant vinyle et l'addition triple de méthylthiol sur de l'acroléine ayant lieu à l'étape a).
